# EUROPÄISCHE PATENTSCHRIFT

(11) **EP 0 816 350 B1**
(45) Veröffentlichungstag und Bekanntmachung des Hinweises auf die Patenterteilung: **07.11.2001**
(21) Anmeldenummer: 97109872.8
(22) Anmeldetag: 17.06.1997
(51) Int. Cl.: C07D 295/02

(54) **Verfahren zur Herstellung von N-substituierten cyclischen Aminen**
Process for the preparation of N-substituted cyclic amines
Procédé de préparation d'amines cycliques substitués sur le nitrogène

(30) Priorität: 18.06.1996 DE 19624283
(43) Veröffentlichungstag der Anmeldung: 07.01.1998
(73) Patentinhaber: BASF AKTIENGESELLSCHAFT, 67056 Ludwigshafen (DE)
(72) Erfinder: Simon, Joachim, Dr., 68161 Mannheim (DE); Dostalek, Roman, Dr., 67354 Römerberg (DE); Lingk, Heinz, 67240 Bobenheim-Roxheim (DE); Becker, Rainer, Dr., 67098 Bad Dürkheim (DE); Henne, Andreas, Dr., 67433 Neustadt (DE); Lebkücher, Rolf, Dr., 68165 Mannheim (DE); Freire Erdbrügger, Cristina, Dr., 67251 Freinsheim (DE); Hesse, Michael, Dr., 67549 Worms (DE); Kratz, Detlef, Dr., 69121 Heidelberg (DE)
(74) Vertreter: Kinzebach, Werner, Dr.

(56) Entgegenhaltungen:
- EP-A- 0 070 397
- EP-A- 0 137 478
- EP-A- 0 440 829
- EP-A- 0 446 783
- WO-A-95/32171
- DE-A- 2 445 303
- GB-A- 1 106 084

## Beschreibung

Die vorliegende Erfindung betrifft ein Verfahren zur Herstellung von N-substituierten cyclischen Aminen durch Umsetzung von Diolen mit primären Aminen an einem kupferhaltigen Katalysator, der durch Tränken eines Trägers mit einer wäßrigen Lösung eines Ammin-Komplexes einer thermisch leicht zersetzbaren Kupferverbindung sowie anschließendes Trocknen und Kalzinieren erhältlich ist und 5 bis 50 Gew.-% Kupfer, berechnet als CuO, enthält.

Verfahren zur Herstellung N-substituierter cyclischer Amine sind bekannt. So können N-methylierte cyclische Amine hergestellt werden, indem man das freie Amin mit Formaldehyd oder Methanol umsetzt (US-A-3 167 551). Dieses Verfahren ist also mehrstufig und erfordert somit einen hohen technischen Aufwand.

Günstiger sind demgegenüber solche Verfahren, bei denen Diole unter Mitwirkung eines Katalysators mit Methylamin zu N-methylsubstituierten cyclischen Aminen umgesetzt werden. Als Katalysatoren kommen hierfür sowohl dehydratisierende Kontakte wie z.B. Phosphorverbindungen in Frage, als auch hydrierend-dehydrierend wirkende Kontakte, z.B. auf Basis von Kupfer, Nickel, Kobalt oder Mischungen, die diese Elemente enthalten.

So wird etwa gemäß der BE-A-842 461 ein Diol diskontinuierlich mit Methylamin in flüssiger Phase in Gegenwart einer Phosphorverbindung zum N-methylierten cyclischen Amin umgesetzt. Hierbei benötigt man hohe Temperaturen (bis 350°C) und sehr hohe Drücke (bis 280 bar), um einigermaßen befriedigende Ausbeuten zu erzielen.

Für die Umsetzung besser geeignete Katalysatoren sind hydrierenddehydrierend wirkende Katalysatoren auf Basis von Kupfer oder Nickel. So beschreibt z.B. die GB-B 1 106 084 die diskontinuierliche Umsetzung von Diethylenglykol mit Monomethylamin an einem CuO/ZnO-Katalysator, die bei 300°C und einem Druck von 100 bis 200 bar in knapp 40%iger Ausbeute N-Methylmorpholin liefert.

Aus der US-A 3 709 881 ist bekannt, daß die Reaktion von Diethylenglykol mit Alkylaminen an einem Nickelkatalysator bei 100 bar und 225 bis 250°C in 20 bis 60%iger Ausbeute zu N-Alkylmorpholin führt.

Ferner ist aus der EP-A 440 829 (= US-A 4 910 304) bekannt, N-substituierte cyclische Amine aus Diolen und Alkylaminen an einem Katalysator, dessen aktive Masse mindestens 80 Gew.-% Kupfer enthält, in Gegenwart einer katalytischen Menge einer basischen Alkali- oder Erdalkaliverbindung herzustellen. Durch kontinuierliche Umsetzung von 1,5-Pentandiol mit Monomethylamin erhält man bei 245°C und 120 bar Wasserstoffpartialdruck bei einem Gesamtdruck von 250 bar in 95%iger Ausbeute N-Methylpiperidin. Aus Diethylenglykol und Monomethylamin entsteht unter ansonsten gleichen Bedingungen das N-Methylmorpholin in 98%iger Ausbeute.

Obwohl diese Ausbeuten technischen Anforderungen genügen, haben die in dieser Anmeldung beschriebenen, hoch kupferhaltigen Katalysatoren den erheblichen Nachteil, daß die anfänglich sehr hohe Aktivität innerhalb weniger Tage fast vollständig verloren geht, wobei der Katalysator zu Kupferschlamm zerfällt (siehe Beispiele). Durch die hohen Kosten, die mit einem häufigen Katalysatorwechsel verbunden sind, wird das Verfahren wirtschaftlich uninteressant.

Soweit das Verfahren mit kupferhaltigen Trägerkatalysatoren durchgeführt wird, werden diese durch Tränken mit Kupfersalzlösungen erhalten. Dadurch werden relativ hohe Calcinierungstemperaturen erforderlich, wodurch die Katalysatorteilchen nachteilig beeinflußt und somit deren Aktivität reduziert wird.

Der Erfindung liegt daher die Aufgabe zugrunde, ein Verfahren zur Herstellung von N-substituierten cyclischen Aminen zur Verfügung zu stellen, das technisch einfach und wirtschaftlich durchführbar ist.

Überraschenderweise wurde nun gefunden, daß diese Aufgabe gelöst wird durch Verwendung eines speziellen Hydrierungs-/Dehydrierungskatalysators, der durch Tränken eines inerten Trägers mit einer wäßrigen Lösung eines Amminkomplexes einer thermisch leicht zersetzbaren Kupferverbindung sowie anschließendes Trocknen und Kalzinieren erhältlich ist und lediglich 5 bis 50 Gew.-% Kupfer, berechnet als CuO, enthält. Der spezielle Katalysator ist aus WO-A-95/32171 als Hydrierungskatalysator für die Herstellung von Alkoholen aus den entsprechenden Carbonylverbindungen bekannt.

Gegenstand der Erfindung ist ein Verfahren zur Herstellung von N-substituierten cyclischen Aminen der allgemeinen Formel I 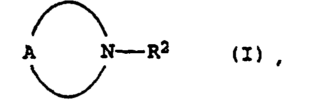 in der
- A: für eine C₄-C₁₂- Alkylengruppe oder eine -(CH₂]ₙ-[O-(CH₂)ₘ]ᵣ-Gruppe, die gegebenenfalls durch einen oder mehrere gleiche oder verschiedene Reste R¹ substituiert sind, und
- R¹ und R²: unabhängig voneinander für C₁-C₃₀- Alkyl, C₂-C₂₀- Alkoxyalkyl, gegebenenfalls durch Alkyl oder Alkoxy substituiertes C₃-C₂₀-Cycloalkyl, C₄-C₂₀- Cycloalkylalkyl, Aryl oder C₇-C₂₀- Arylalkyl stehen,
- n,m: unabhängig voneinander für eine Zahl im Bereich von 2 bis 8 stehen und
- r: für eine Zahl im Bereich von 1 bis 3 steht,
durch Umsetzung von primären Aminen der allgemeinen Formel II

R²―NH₂ (II)

mit einem Diol der allgemeinen Formel III

HO―A―OH (III),

wobei R² und A die oben genannten Bedeutungen haben, in Anwesenheit von Wasserstoff und eines kupferhaltigen Katalysators, das dadurch gekennzeichnet ist, daß man einen Katalysator verwendet, der erhältlich ist durch Tränken eines inerten Trägers mit einer wäßrigen Lösung eines Amminkomplexes einer Kupferverbindung, die sich bei Koltinierungstemperaturen von 200 bis 400°C zu oxidischen Kupferverbindungen oder elementarem Kupfer zersetzt sowie anschließendes Trocknen und Kalzinieren, und der 5 bis 50 Gew.-% Kupfer, berechnet als CuO, bezogen auf das Gesamtgewicht des kalzinierten Katalysators, enthält.

Die Substituenten in den Formeln I bis III haben folgende Bedeutung:
A bedeutet:
   - eine C₄-C₁₂-Alkylengruppe, z.B. -(CH₂)₄-, -(CH₂)₅, -(CH₂)₆-, -(CH₂)₇-, -(CH₂)₈-, -(CH₂)₉-, -(CH₂)₁₀-, -(CH₂)₁₁-, -(CH₂)₁₂-, wobei C₄-C₈-Alkylengruppen bevorzugt sind,
   - eine -(CH₂)ₙ-[O-(CH₂)ₘ]ᵣ-Gruppe, in der n und m unabhängig voneinander für 2 bis 8 stehen und r für 1 bis 3 steht, wobei die -(CH₂)ₙ-[O-(CH₂)ₘ]ᵣ-Gruppe vorzugsweise aus 5 bis 12 Gliedern besteht, wobei vorzugsweise n und m unabhängig voneinander für 2 bis 4 stehen und r für 1 bis 2 steht, wie -(CH₂)₂-O-(CH₂)₂-, -(CH₂)₃-O-(CH₂)₂-, -(CH₂)₃-O-(CH₂)₃-, -(CH₂)₄-O-(CH₂)₂-, -(CH₂)₄-O-(CH₂)₃-, -(CH₂)₄-O-(CH₂)₄- und -(CH₂)₂-O-(CH₂)₂-O-(CH₂)₂-.
   A kann durch einen oder mehrere Reste R¹ substituiert sein, wobei die Reste R¹ in einer definierten Verbindung nicht gleich sein müssen. Bevorzugt sind 1 bis 6 Reste R¹, besonders bevorzugt 1 bis 4.
R¹ und R² bedeuten unabhängig voneinander:
   - unverzweigtes oder verzweigtes C₁-C₃₀-Alkyl, bevorzugt unverzweigtes oder verzweigtes C₁- bis C₂₀-Alkyl, besonders bevorzugt unverzweigtes oder verzweigtes C₁- bis C₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl.
   - C₂- bis C₂₀-Alkoxyalkyl, wie Methoxymethyl, Ethoxypropyl und Butoxydecyl,
   - C₃- bis C₂₀-Cycloalkyl, besonders bevorzugt C₃- bis C₈-Cycloalkyl, wie Cyclopropyl, Cyclobutyl, Cyclopentyl, Cyclohexyl, Cycloheptyl und Cyclooctyl,
   - C₄- bis C₂₀-Alkylcycloalkyl, wobei die Cycloalkylgruppe 3 bis 8, vorzugsweise 3 bis 6 Kohlenstoffatome aufweist, wie Methylcyclopropyl, Isopropylcyclohexyl und Dodecylcyclohexyl,
   - C₄- bis C₂₀-Cycloalkylalkyl, wobei die Alkylgruppe 1 bis 14, vorzugsweise 1 bis 10 Kohlenstoffatome aufweist, wie Cyclopropylmethyl, Cyclopentylmethyl, Cyclohexylmethyl, Cyclopropylethyl, Cyclopentylethyl und Cyclohexylethyl,
   - Aryl, wie Phenyl, 1-Naphthyl, 2-Naphthyl, 1-Anthranyl, 2-Anthranyl und 9-Anthranyl, bevorzugt Phenyl,
   - C₇- bis C₂₀-Arylalkyl, besonders bevorzugt C₇- bis C₂₀-Phenylalkyl, wie Benzyl, Phenethyl oder Phenyldecyl.

Endprodukte der Formel I sind beispielsweise:
N-Methylpyrrolidin, N-Ethylpyrrolidin, N-Methylpiperidin,
N-Ethylpiperidin, N-Methylmorpholin, 1-Methyl-1-azacycloundecan und N-n-Butylmorpholin. Besonders bevorzugt ist das Verfahren zur Herstellung von N-methylsubstituierten cyclischen Aminverbindungen.

Ausgangsstoffe der Formel II sind beispielsweise: Methylamin, Ethylamin, n-Propylamin, iso-Propylamin, n-Butylamin, Isobutylamin, n-Dodecylamin, Anilin und p-Toluidin.

Ausgangsstoffe der Formel III sind beispielsweise:
Butan-1,4-diol,
3-Methylpentan-1,5-diol,
Diethylenglykol(3-Oxapentan-1,5-diol),
Dibutylenglykol(5-Oxanonan-1,9-diol),
Triethylenglykol(3,5-Dioxaheptan-1,7-diol),
1,3-Dipropylenglykol(4-Oxaheptan-1-diol),
Dipropylenglykol(1,5-Dimethyl-3-Oxapentan-1,5-diol).
5-Oxanonan-1,9-diol und
Hexan-2,5-diol.

Allgemein eignen sich als Diole III solche mit primären oder sekundären Hydroxylgruppen, besonders solche mit primären Hydroxylgruppen, also α,ω-Diole.

Die Reaktion verläuft gemäß der unten aufgeführten allgemeinen Reaktionsgleichung [1]

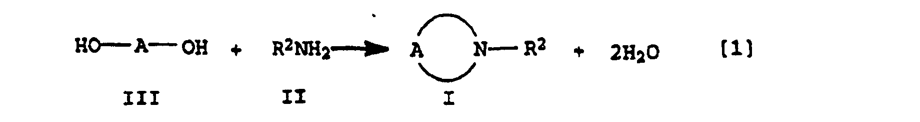

Die Reaktion wird in Gegenwart von Wasserstoff diskontinuierlich oder vorzugsweise kontinuierlich in der Flüssigphase bei einem Druck von 100 bis 300 bar, bevorzugt 200 bis 250 bar und Temperaturen von 200 bis 300°C, bevorzugt 220 bis 280°C, oder in der Gasphase bei einem Druck von 1 bis 50 bar, bevorzugt 10 bis 30 bar und Temperaturen von 200 bis 300°C, bevorzugt 200 bis 250°C durchgeführt. Die Flüssigphasenreaktion kann als Suspensions-, Riesel- oder Sumpfreaktion durchgeführt werden. Es empfiehlt sich. schwer flüchtige oder feste Monoalkylamine II oder Diole III in einem inerten Lösungsmittel gelöst zu verwenden, wobei pro Mol II bzw. III 50 bis 200 ml, vorzugsweise 100 bis 150 ml eines inerten Lösungsmittels in der Regel genügen.

Als inerte Lösungsmittel eignen sich Ether wie Diethylether, Methyl-isopropylether, Methyl-tert.-butylether, Tetrahydrofuran und Dioxan; aliphatische Kohlenwasserstoffe wie n-Pentan, das Pentanisomerengemisch, n-Hexan, das Hexanisomerengemisch, Petrolether und Cyclohexan, aromatische Kohlenwasserstoffe wie Benzol, Toluol, die Xylole und deren Isomerengemisch oder Mischungen dieser Lösungsmittel.

Bei der diskontinuierlichen Betriebsweise verwendet man den Katalysator vorzugsweise in Form einer feinteiligen Suspension. Die eingesetzte Katalysatormenge entspricht 3 bis 30 g, vorzugsweise 10 bis 15 g Kupfer pro kg Diol. Das Diol III kann mit dem darin suspendierten Katalysator in Wasser vorgelegt und das Amin II wie für die Reaktion erforderlich bei der Reaktionstemperatur unter den gewählten Bedingungen allmählich hinzugegeben werden. Beim Einsatz der Reaktanden in äquimolaren Mengen können die Komponenten Diol, Amin und Wasser auch gemeinsam vorgelegt werden.

Bei der kontinuierlichen Verfahrensweise wird der Katalysator als Festbett in Form von Perlen, Strängen, Tabletten oder Ringen eingesetzt. Die Menge an Katalysator liegt im Bereich von 100 bis 1000, vorzugsweise von 200 bis 400 g Kupfer pro kg Diol pro Stunde. Besonders bevorzugt sind Festbettkontakte, die durch Rieselfahrweise oder durch Sumpffahrweise, bei der die gesamte Katalysatorschicht zusammenhängend mit Flüssigkeit bedeckt sein sollte, betrieben werden. Bei dieser Ausführungsform werden dann das Diol III und das Amin II im gewählten Molverhältnis über das Katalysatorfestbett geführt.

Das Molverhältnis von Monoalkylamin zum Diol liegt im allgemeinen im Bereich von 1:1 bis 2:1, bevorzugt 1,1:1 bis 1,5:1.

Brauchbare Hydrierungs-/Dehydrierungskatalysatoren sind in der WO-A-95/32171 beschrieben, auf deren Inhalt hiermit in vollem Umfang Bezug genommen wird. Sie können demnach in verschiedener Weise hergestellt werden. Besonders bevorzugt ist jedoch die Tränkung eines Trägers für 2 bis 60, vorzugsweise für 5 bis 30 Minuten mit einer überstehenden wäßrigen Lösung einer thermisch leicht zersetzbaren Kupferverbindung, anschließende Trocknung und Kalzinierung bei Temperaturen von 200 bis 400°C, vorzugsweise von 250 bis 350°C, besonders bevorzugt bei 300°C.

Die Tränkung mit einer überstehenden Lösung führt zu einer relativ gleichmäßigen Durchtränkung des Trägermaterials und damit zu einer feineren und gleichmäßigeren Verteilung des Kupfers im bzw. auf dem Träger. Die Ausbildung von ungleichförmigen Konzentrationsprofilen über den Querschnitt des Trägermaterials wie sie bei anderen Tränkungsmethoden auftreten können, werden bei diesem Verfahren vermieden, wodurch die Herstellung des erfindungsgemäß zu verwendenden Katalysators reproduzierbarer und folglich wirtschaftlicher wird.

Brauchbare Trägermaterialien sind die üblicherweise verwendeten wie z.B. SiO₂, Silikate, Bimsstein, Diatomeenerde, Kieselgel, Aluminiumoxid, Zeolithe und Zirkoniumdioxid, wobei SiO₂-haltige Träger besonders bevorzugt sind. Das für den Katalysator verwendete Trägermaterial wird als SiO₂-haltig bezeichnet, wenn es SiO₂ oder ein Silikat wie etwa Magnesiumsilikat enthält. Da die anionischen Silikatgruppen in monomerer, oligomerer und polymerer Form nebeneinander im Katalysator vorliegen, werden sie analytisch als SiO₂ erfaßt und berechnet.

Unter thermisch leicht zersetzbaren Kupferverbindungen werden Kupferverbindungen verstanden, die sich bei Calciniertemperaturen von 200 bis 400°C, vorzugsweise von 250 bis 350°C, zu oxidischen Kupferverbindungen oder elementarem Kupfer zersetzen. Beispielhaft für solche Verbindungen seien Kupfercarbonat, Kupferoxalat oder Kupferformiat genannt. Unter Kupfercarbonat wird im Rahmen der vorliegenden Erfindung das basische Kupfercarbonat verstanden. Es läßt sich durch die allgemeine Formel a CuCO₃ · b Cu(OH)₂ · c H₂0 mit a,b,c ∈ N beschreiben und kann z.B. durch Fällung von Kupfer(II)-salzen mit Sodalösung erhalten werden. Man verwendet diese Kupfersalze in Form ihrer relativ stabilen, gut wasserlöslichen Ammin-Komplexe. Dazu zählen Komplexe mit Ammoniak oder Aminen. Besonders bevorzugt wird hierzu ammoniakalische Kupfercarbonat-Lösung verwendet. Die Verwendung dieser thermisch leicht zersetzbaren Kupferverbindungen ermöglicht die Calcinierung bei den genannten niedrigen Temperaturen, während herkömmlicherweise zur Tränkung eingesetzte Kupfersalze, wie Kupfernitrat oder Kupfersulfat, im allgemeinen Calciniertemperaturen um 500°C erfordern. Sowohl die Verwendung thermisch leicht zersetzbarer Kupferverbindungen als auch die Anwendung niedriger Calciniertemperaturen ermöglicht die Erzeugung kleiner, gleichmäßig verteilter Kupferkristallite und damit einer höheren katalytisch wirksamen Kupferoberfläche im fertigen Katalysator.

Die vorteilhafte Auswirkung dieser vergrößerten katalytisch wirksamen Kupferoberfläche wird unter anderem durch den Vergleich der auf S. 8f aufgeführten Beispiele 1.1 (erfindungsgemäßes Verfahren) und 1.4 (Stand der Technik, Katalysator erhalten durch Tränken mit Kupfernitrat-Lösung und Kalzinieren bei 500°C) deutlich. Das erfindungsgemäße Verfahren liefert deutlich höhere Ausbeuten als das dem Stand der Technik entsprechende.

Ein Maß für die wirksame Kupferoberfläche bei einer bestimmten Menge an eingesetztem Kupfer ist die Dispersion. Sie ist definiert als Verhältnis der Zahl der Kupfer-Atome an der Oberfläche der Kupfer-Kristallite zur Gesamtzahl der Kupfer-Atome in den Kupfer-Kristalliten des Trägers.

Die Dispersion des Kupfers in einem Katalysator kann direkt aus der Größe der Kristallite oder indirekt aus der zur Oxidation der Kupferoberfläche nötigen Menge an Sauerstoff und der bei der Tränkung verwendeten Menge an Kupfer bestimmt werden.

Neben einer erhöhten Dispersion weisen die nach WO-A-95/32171 hergestellten Katalysatoren aber auch eine verbesserte mechanische Stabilität auf. Kennzeichnend für die erhöhte mechanische Stabilität sind vor allem eine größere Härte und ein verminderter Abrieb des Katalysators.

Der Kupferkatalysator enthält 5 bis 50, vorzugsweise 15 bis 40, besonders bevorzugt 20 bis 30 Gew.-% Kupfer, berechnet als CuO, bezogen auf das Gesamtgewicht des kalzinierten Katalysators. Er enthält außerdem 50 bis 95, vorzugsweise 60 bis 85, besonders bevorzugt 70 bis 80 Gew.-% Silicium, berechnet als SiO₂, bezogen auf das Gesamtgewicht des kalzinierten Katalysators.

Der Katalysator wird vorzugsweise in situ mit Wasserstoff reduziert und so in die aktive Form überführt. Besonders bevorzugt ist ein Katalysator, der in diesem Zustand eine Kupferoberfläche von > 20 m²/g, eine Teilchengröße < 100 nm und eine Cu-Dispersion von > 20% aufweist.

Nach der Umsetzung werden die Produkte durch übliche Verfahren, z.B. durch Destillation oder Extraktion aus dem Reaktionsgemisch isoliert; nicht umgesetzte Ausgangsstoffe werden gegebenenfalls in die Umsetzung zurückgeführt.

Die folgenden Beispiele dienen der Erläuterung des erfindungsgemäßen Verfahrens, ohne dieses darauf zu beschränken.

### Beispiele:

### Herstellung des Katalysators:

Der Katalysator wird durch Tränken von 1 l (0,45 kg) SiO₂-Kugeln von 3 bis 5 mm Durchmesser bei Raumtemperatur mit 1 l 35%iger, wäßriger Ammoniaklösung, in der zuvor 151 g basisches Kupfercarbonat (berechnet als 2CuCO₃·Cu(OH)₂) gelöst worden ist, erhalten. Die Tränkung erfolgt in überstehender Lösung während 15 Minuten.

Die getränkten Kugeln werden 5 Stunden bei 120°C getrocknet und danach 2 Stunden bei 300°C kalziniert. Diese Tränk- und Kalzinierschritte werden einmal wiederholt und ergeben 1 l (0,62 kg) fertigen Katalysator, der 30 Gew.-% CuO auf 70 Gew.-% SiO₂ enthält.

### 1. Herstellung von N-Methylmorpholin:

### Beispiel 1.1:

Durch einen Rohrreaktor mit einem Innendurchmesser von 3,2 cm und einer Höhe von 125 cm, der eine Schüttung von 700 ml des oben beschriebenen und mit Wasserstoff reduzierten Katalysators in Form von 3 x 5 mm Perlen enthält, werden in Rieselfahrweise stündlich 80 ml Monomethylamin und 210 ml Diethylenglykol bei 280°C, 200 bar Gesamtdruck und 100 bar Wasserstoffpartialdruck geleitet. Nach einer Laufzeit von 14 1/2 Tagen beträgt die Ausbeute an N-Methylmorpholin 82% der Theorie. Der ausgebaute Katalysator zeigt keine mechanischen Veränderungen.

### Beispiel 1.2:

Durch einen Rohrreaktor mit einem Innendurchmesser von 4,1 cm und einer Höhe von 350 cm, der eine Schüttung von 1000 ml des oben beschriebenen und mit Wasserstoff reduzierten Katalysators in Form von 3 x 5 mm Perlen enthält, werden in Gasphasenfahrweise stündlich 130 ml Monomethylamin, 200 ml Diethylenglykol und 300 ml Wasserstoff bei 200°C und 20 bar Gesamtdruck geleitet. Nach einer Laufzeit von 4 1/2 Tagen beträgt die Ausbeute an N-Methylmorpholin 11% der Theorie. Der ausgebaute Katalysator zeigt keine mechanischen Veränderungen.

### Beispiel 1.3: (Stand der Technik)

Durch einen Rohrreaktor mit einem Innendurchmesser von 3,2 cm und einer Höhe von 125 cm, der eine Schüttung von 700 ml des in der EP-A 440 829 auf S. 4, Z 16-20 beschriebenen und mit Wasserstoff reduzierten Katalysators in Form von 3 x 5 mm Tabletten enthält, werden in Rieselfahrweise stündlich 80 ml Monomethylamin und 210 ml Diethylenglykol bei 280°C, 200 bar Gesamtdruck und 100 bar Wasserstoffpartialdruck geleitet. Nach einer Laufzeit von 14 1/2 Tagen beträgt die Ausbeute an N-Methylmorpholin 69% der Theorie. Der ausgebaute Katalysator ist vollständig zu rotem Schlamm zerfallen.

### Beispiel 1.4: (Stand der Technik, Tränkkatalysatoren, Vergleich zu Beispiel 1.1)

### Herstellung des Katalysators

Der Katalysator wird nach bekannter Literaturvorschrift (siehe z.B. Berkman, Morrell und Egloff, "Catalysis", 1940) durch Tränken von γ-Al₂O₃ mit einer Kupfernitrat-Lösung und anschließendes Kalzinieren bei 500°C erhalten. Der fertige Katalysator enthält 16% CuO.

### Herstellung von N-Methylmorpholin

Durch einen Rohrreaktor mit einem Innendurchmesser von 3,2 cm und einer Höhe von 125 cm, der eine Schüttung von 700 ml des oben beschriebenen Katalysators in Form von 4 mm-Strängen enthält, werden in Rieselfahrweise stündlich 80 ml Monomethylamin und 210 ml Diethylenglykol bei 280°C, 200 bar Gesamtdruck und 100 bar Wasserstoffpartialdruck geleitet. Nach einer Laufzeit von 10 Tagen beträgt die Ausbeute an N-Methylmorpholin knapp 35% der Theorie.

Der ausgebaute Katalysator zeigt keine mechanischen Veränderungen.

### 2. Herstellung von N-Methylpiperidin:

### Beispiel 2.1:

Durch einen Rohrreaktor mit einem Innendurchmesser von 3,2 cm und einer Höhe von 125 cm, der eine Schüttung von 700 ml des in Beispiel 1.1 eingesetzten und mit Wasserstoff reduzierten Katalysators in Form von 3 x 5 mm Perlen enthält, werden in Sumpffahrweise stündlich 130 ml Monomethylamin und 210 ml Pentandiol-1,5 bei 240°C, 200 bar Gesamtdruck und 70 bar Wasserstoffpartialdruck geleitet. Nach einer Laufzeit von 40 Tagen beträgt die Ausbeute an N-Methylpiperidin 82% der Theorie. Der ausgebaute Katalysator zeigt keine mechanischen Veränderungen.

### Beispiel 2.2: (Stand der Technik)

Durch einen Rohrreaktor mit einem Innendurchmesser von 3,2 cm und einer Höhe von 125 cm, der eine Schüttung von 700 ml des in der EP-A 440 829 auf S. 4, Z 16-20 beschriebenen und mit Wasserstoff reduzierten Katalysators in Form von 3 x 5 mm Tabletten enthält, werden in Sumpffahrweise stündlich 120 ml Monomethylamin und 210 ml Pentandiol-1,5 bei 240°C, 200 bar Gesamtdruck und 80 bar Wasserstoffpartialdruck geleitet. Nach einer Laufzeit von 10 Tagen beträgt die Ausbeute an N-Methylpiperidin 62% der Theorie. Der ausgebaute Katalysator ist vollständig zu rotem Schlamm zerfallen.

## Patentansprüche

1. Verfahren zur Herstellung von N-substituierten cyclischen Aminen der allgemeinen Formel I 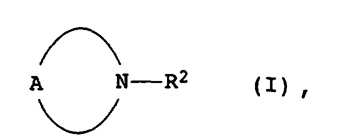 in der
A für eine C₄-C₁₂-Alkylengruppe oder eine -(CH₂)ₙ-[O-(CH₂)ₘ]ᵣ-Gruppe, die gegebenenfalls durch einen oder mehrere gleiche oder verschiedene Reste R¹ substituiert sind, und
R¹ und R² unabhängig voneinander für C₁-C₃₀-Alkyl, C₂-C₂₀-Alkoxyalkyl, gegebenenfalls durch Alkyl oder Alkoxy substituiertes C₃-C₂₀-Cycloalkyl, C₄-C₂₀-Cycloalkylalkyl, Aryl oder C₇-C₂₀-Arylalkyl stehen,
n,m unabhängig voneinander für eine Zahl im Bereich von 2 bis 8 stehen und
r für eine Zahl im Bereich von 1 bis 3 steht,
durch Umsetzung von primären Aminen der allgemeinen Formel II
R²―NH₂ (II)
mit einem Diol der allgemeinen Formel III
HO―A―OH (III),
wobei R² und A die oben genannten Bedeutungen haben, in Anwesenheit von Wasserstoff und eines kupferhaltigen Katalysators, **dadurch gekennzeichnet, daß** man einen Katalysator verwendet, der erhältlich ist durch Tränken eines inerten Trägers mit einer wäßrigen Lösung eines Amminkomplexes einer Kupferverbindung, die sich bei Kalzinierungstemperaturen von 200 bis 400 °C zu oxidischen Kupferverbindungen oder elementarem Kupfer zersetzt sowie anschließendes Trocknen und Kalzinieren, und der 5 bis 50 Gew.-% Kupfer, berechnet als CuO, bezogen auf das Gesamtgewicht des kalzinierten Katalysators, enthält.

2. Verfahren nach Anspruch 1, **dadurch gekennzeichnet, daß** man als Kupferverbindung Kupfercarbonat, Kupferoxalat und/oder Kupferformiat verwendet.

3. Verfahren nach Anspruch 1 oder 2, **dadurch gekennzeichnet, daß** man einen Katalysator verwendet, der 20 bis 30 Gew.-% Kupfer, berechnet als CuO, bezogen auf das Gesamtgewicht des kalzinierten Katalysators, enthält.

4. Verfahren nach einem der Ansprüche 1 bis 3, **dadurch gekennzeichnet, daß** man die Reaktion in flüssiger Phase bei einem Druck von 100 bis 300 bar und einer Temperatur von 200 bis 300 °C durchführt.

5. Verfahren nach Anspruch 4, **dadurch gekennzeichnet, daß** man die Reaktion bei 200 bis 250 bar und einer Temperatur von 220 bis 280 °C durchführt.

6. Verfahren nach einem der Ansprüche 1 bis 3, **dadurch gekennzeichnet, daß** man die Reaktion in der Gasphase bei einem Druck von 1 bis 50 bar und einer Temperatur von 200 bis 300 °C durchführt.

7. Verfahren nach Anspruch 6, **dadurch gekennzeichnet, dass** man die Reaktion bei 10 bis 30 bar und einer Temperatur von 200 bis 250 °C durchführt.

8. Verfahren nach einem der Ansprüche 1 bis 7, **dadurch gekennzeichnet, daß** man die Umsetzung kontinuierlich durchführt.

9. Verwendung eines wie in einem der Ansprüche 1 bis 3 definierten kupferhaltigen Katalysators zur Herstellung von N-substituierten cyclischen Aminen der in Anspruch 1 definierten allgemeinen Formel I.

## Claims

1. A process for preparing N-substituted cyclic amines of the formula I 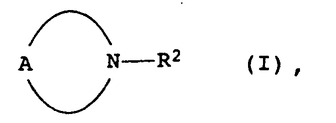 where
A is a C₄-C₁₂-alkylene group or a -(CH₂)ₙ-[O-(CH₂)ₘ]ᵣ- group which may each be substituted by one or more identical or different radicals R¹, and
R¹ and R² are, independently of one another, C₁-C₃₀-alkyl, C₂-C₂₀-alkoxyalkyl, unsubstituted or alkyl- or alkoxy-substituted C₃-C₂₀-cycloalkyl, C₄-C₂₀-cycloalkylalkyl, aryl or C₇-C₂₀-arylalkyl,
n,m are each, independently of one another, a number in the range from 2 to 8 and
r is a number in the range from 1 to 3,
by reacting primary amines of the general formula II
R²―NH₂ (II)
with a diol of the general formula III
HO―A―OH (III),
where R² and A are as defined above, in the presence of hydrogen and a copper-containing catalyst, wherein the catalyst used is obtainable by impregnation of an inert support with an aqueous solution of an ammine complex of a copper compound which decomposes at calcination temperatures of from 200 to 400°C to give oxidic copper compounds or elemental copper, and subsequent drying and calcination and contains from 5 to 50% by weight of copper, calculated as CuO, based on the total weight of the calcined catalyst.

2. A process as claimed in claim 1, wherein the copper compound used is copper carbonate, copper oxalate and/or copper formate.

3. A process as claimed in claim 1 or 2, wherein the catalyst used contains from 20 to 30% by weight of copper, calculated as CuO, based on the total weight of the calcined catalyst.

4. A process as claimed in any of claims 1 to 3, wherein the reaction is carried out in the liquid phase at from 200 to 300°C and a pressure of from 100 to 300 bar.

5. A process as claimed in claim 4, wherein the reaction is carried out at from 220 to 280°C and from 200 to 250 bar.

6. A process as claimed in any of claims 1 to 3, wherein the reaction is carried out in the gas phase at from 200 to 300°C and a pressure of from 1 to 50 bar.

7. A process as claimed in claim 6, wherein the reaction is carried out at from 200 to 250°C and from 10 to 30 bar.

8. A process as claimed in any of claims 1 to 7, wherein the reaction is carried out continuously.

9. Use of a copper-containing catalyst as defined in any of claims 1 to 3 for preparing N-substituted cyclic amines of the general formula I defined in claim 1.

## Revendications

1. Procédé de préparation d'amines cycliques N-substituées de formule générale I 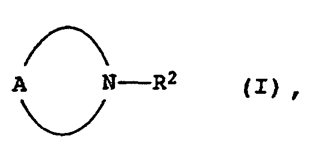 dans laquelle
A est un groupe alkylène en C₄-C₁₂ ou un groupe -(CH₂)ₙ-[O-(CH₂)ₘ]ᵣ, qui est éventuellement substitué par un ou plusieurs radicaux R¹ identiques ou différents, et
R¹ et R² représentent chacun indépendamment de l'autre un groupe alkyle en C₁-C₃₀, alcoxyalkyle en C₂-C₂₀, ou encore cycloalkyle en C₃-C₂₀, cycloalkylalkyle en C₄-C₂₀, aryle ou arylalkyle en C₇-C₂₀ éventuellement substitué par des substituants alkyle ou alcoxy,
n et m représentent chacun indépendamment de l'autre un nombre compris entre 2 et 8, et
r est un nombre compris entre 1 et 3,
par réaction d'amines primaires de formule générale II
R²-NH₂ (II)
avec un diol de formule générale III
HO-A-OH (III)
où R² et A ont les significations données ci-dessus, en présence d'hydrogène et d'un catalyseur contenant du cuivre, **caractérisé en ce qu'**on utilise un catalyseur pouvant être obtenu par imprégnation d'un support inerte par une solution aqueuse d'un complexe ammine d'un composé du cuivre, qui à des températures de calcination de 200 à 400°C se décompose en des composés oxydés du cuivre ou en cuivre élémentaire, puis par séchage et calcination, et qui contient 5 à 50 % en poids de cuivre, calculé en CuO, par rapport au poids total du catalyseur calciné.

2. Procédé selon la revendication 1, **caractérisé en ce qu'**on utilise en tant que composé du cuivre le carbonate de cuivre, l'oxalate de cuivre et/ou le formiate de cuivre.

3. Procédé selon la revendication 1 ou 2, **caractérisé en ce qu'**on utilise un catalyseur qui contient de 20 à 30 % en poids de cuivre, calculé en CuO, par rapport au poids total du catalyseur calciné.

4. Procédé selon l'une des revendications 1 à 3, **caractérisé en ce qu'**on met en oeuvre la réaction en phase liquide sous une pression de 100 à 300 bar et à une température de 200 à 300°C.

5. Procédé selon la revendication 4, **caractérisé en ce qu'**on met en oeuvre la réaction sous une pression de 200 à 250 bar et à une température de 220 à 280°C.

6. Procédé selon l'une des revendications 1 à 3, **caractérisé en ce qu'**on met en oeuvre la réaction en phase gazeuse sous une pression de 1 à 50 bar et à une température de 200 à 300°C.

7. Procédé selon la revendication 6, **caractérisé en ce qu'**on met en oeuvre la réaction sous une pression de 10 à 30 bar et à une température de 200 à 250°C.

8. Procédé selon l'une des revendications 1 à 7, **caractérisé en ce qu'**on met en oeuvre la réaction d'une manière continue.

9. Utilisation d'un catalyseur contenant du cuivre, selon l'une des revendications 1 à 3, pour préparer des amines cycliques N-substituées ayant la formule générale I donnée dans la revendication 1.
